# EUROPEAN PATENT APPLICATION

(11) **EP 1 405 622 A1**
(43) Date of publication of application: **07.04.2004**
(21) Application number: 02743834.0
(22) Date of filing: 04.07.2002
(51) Int. Cl.: A61J 1/00, B31B 1/84, B65D 30/10

(54) **MEDICAL CONNECTED CONTAINER ASSEMBLY AND METHOD OF PRODUCING THE SAME**

(30) Priority: 06.07.2001 JP 2001205623
(71) Applicant: Showa Denko Plastic Products Co. Ltd., Tokyo 103-0012 (JP)
(72) Inventor: MIZUO, Takayuki c/o Showa Denko Plastic, Chuo-ku Tokyo 103-0012 (JP); NAKAGAWA, Teruaki c/o Showa Denko K. K., Kawasaki-shi Kanagawa 210-0858 (JP)
(74) Representative: Strehl Schübel-Hopf & Partner
(86) International application number: PCT/JP2002/006803
(87) International publication number: WO 2003/003969

(57) **Abstract**

Two continuous films are sealed with each other by melting, bag type compartments are continuously formed, and a port member is provided at one side or both sides of side edges of each bag type compartment, and simultaneously, a relaxation means such as a notch at a jointing part of compartments or a connection part are provided to release tension caused by melt sealing of the adjacent port members in connected state, and thereby, a flat type connected container is provided.

## Description

### TECHNICAL FIELD

The present invention relates to medical containers, such as transfusion fluid bags or CAPD (continuous ambulatory peritoneal dialysis) bags, which are filled with blood or a medicinal solution, and is employed in that form without further modification. In particular, the present invention relates to connected containers for medical use in which a plurality of individual containers are connected together, these connected containers being easy to handle during manufacture, packaging, transport, and filling, and able to prevent damage to the bag contents during storage and transportation.

This application is based on Japanese Patent Application No. 2001-205623, the content of which is incorporated herein by reference.

### BACKGROUND ART

In the past, resin bottles formed using a blow molding method came to be employed in place of glass bottles as medical containers such as transfusion fluid bags or CAPD bags. Currently, there is a shift to an arrangement in which plastic film is sealed and formed into the shape of a bag, and a port member is attached to the bag by melting. Since these bag type medical containers have high pliability, and the volume of each container automatically reduced as the contained fluid is discharged, secondary air introduction is not required. As a result, introduction of contaminants such as microscopic dust particles or bacteria suspended in room air can be prevented. In addition, since containers of this type significantly reduce the amount and weight of waste, it is thought that they will be increasingly employed in the future.

Bag type medical containers described above may be sealed so as to be bag type compartments in a continuous piece of film except sealing at the port member. This process for forming the bag is highly efficient, the required machinery is simple and requires little space, and productivity is high. In general, however, the port member is not attached until the bags have been formed and cut apart into sheets.

Proposals have been made which improve an embodiment of a connected container that is produced by a process that is continuous through the attachment of the port member (Japanese Unexamined Patent Application, First Publication No. 2001-112847). However, in this case as well, it is necessary to cut free each container after attaching the port member to the container, and supply the containers one at a time to a contents filling equipment.

Therefore, a large and complicated mechanical structure is required in order to position and fix each bag type container cut free in place during delivery, storage, relay, and contents filling process since the container is pliable, and for storage, packaging, and transport of the container after filling. It is thus difficult to respond if changes are made in the dimensions of the container and adjustments in the devices are difficult. In addition, because maintaining the machinery is troublesome and breakdowns occur, manufacturing costs tend to rise.

Furthermore, the bag-forming process and the filling process are independent processes. In addition, since the container is pliable, it does not have a fixed shape. Thus, relay, packing, and feeding to the contents filling equipment is difficult. In addition, since the container itself is not self-supporting, it is difficult to hold its shape. As a result, when the containers are stacked, problems can readily occur such as a load shift, deformation, scratch, and the like, so that troublesome special handling is needed. Thus, it is often the case that the volume-to-container capacity remarkably increases, and efficiency during the production process is poor.

### DISCLOSURE OF INVENTION

It is an aim of the present invention to provide a multiple connected medical containers which are easily stored, packaged, and transported in the unfilled state, are readily supplied in a continuous manner from their packing to the contents filling equipment in the contents filling process, and for which packaging, storage, taking out, and separation into individual bags after filling is carried out easily and certainly. More specifically, the present invention provides bag type multiple connected medical containers in which, in the container forming process, port members can be attached by melting continuously from the film, the tension caused by the melting of the port member is released, and the containers can be handled as a flat connected unit.

A first embodiment of the present invention provides multiple connected medical containers comprising: a continuous film having a melted sealed part; bag type compartments which are continuously provided by the melted sealed part; a port member which is provided at one side or both sides of side edges of each bag type compartment; and a relaxation means for releasing stress or tension at side edges of the continuous film.

The relaxation means may be a jointing part of compartments or a connection part, each having a notch.

The notch may be a slit, a V-shaped notch, or a U-shaped notch.

The relaxation means may release stress or tension by heating the side edges before or after providing the port member.

The relaxation means may be a connection part which is provided between adjacent jointing parts of compartments without melting and sealing.

The multiple connected medical containers according to the first embodiment may comprise a folding seam on the jointing part of compartments or the connection part so that every set member of bag type compartments is folded without the port members stacking on each other.

The joining part of compartments may have a width greater than a maximum diameter of the port member.

The port members may be disposed toward the side edge of the bag type compartment s that every set number of bag type compartments is folded without the port members stacking on each other.

The port members may be provided on alternating sides after every set number of the port members to the number of folds so that every set number of bag type compartments are folded without the port members stacking on each other.

The folding seam may have perforations, notches, or discontinuous slits provided on the jointing part of compartments or the connection part.

The continuous film may be in a form of tubular, a laminate of two films, or a two stacked layers film provided by folding a film having a wide width.

The continuous film may be a plastic film being a single layer or a laminate with a thickness of 100 ∼ 500 µm.

The continuous film may contain joining faces made by a melt sealable resin.

The multiple connected medical containers according to the first embodiment may be used for continuous transfusion fluid bags or CAPD bags.

The multiple connected medical containers according to the first embodiment may comprise a hole for suspension provided at the melted sealed part of either port member.

A second embodiment of the present invention provides a packing method of multiple connected medical containers, comprising a step of: folding the continuous multiple connected medical containers according to the first embodiment by a set number of two or more bag type compartments without the port members stacking on each other.

A third embodiment of the present invention provides a packaging method of multiple connected medical containers, comprising a step of: packing the multiple connected medical containers so that compartments at a starting end and a finishing end in the multiple connected medical containers are taken out of the packaging unit, when the multiple connected medical containers according to claim 1 are packed into a packaging case (packaging unit) while being folded from a bottom of the case in order.

A fourth embodiment of the present invention provides a manufacturing method of multiple connected medical containers, comprising steps of: supplying two stacked pieces of a film to a melt sealer; sealing by melting a jointing part of a compartment except for an attachment part of a port member; pulling apart one or both side edges of the film where the attachment part of a port member is provided; sealing by melting the port member; and providing a notch at the side edges of the jointing part of compartments either before or after sealing by melting the port member.

A fifth embodiment of the present invention relates to a manufacturing method of multiple connected medical containers, comprising steps of: supplying two stacked pieces of a film to a melt sealer; sealing by melting a jointing part of a compartment except for an attachment part of a port member; pulling apart one or both side edges of the film where the attachment part of a port member is provided; forming multiple connected containers by melting and sealing the port member; and heating the side edges or the entirety of the containers to relieve tension.

A sixth embodiment of the present invention provides a contents filling method of multiple connected medical containers, comprising steps of: supplying the multiple connected medical containers according to the first embodiment to a contents filling device continuously; and filling contents into the multiple connected medical containers.

### BRIEF DESCRIPTION OF DRAWINGS

Fig. 1 shows multiple connected medical containers according to the present invention (without connection parts) in which port members are provided at both sides of side edges.
Fig. 2 is a conceptual view in which the multiple connected medical containers shown in Fig. 1 are folded.
Fig. 3 shows the way to continuously take out packaged multiple connected medical containers.
Fig. 4 shows multiple connected medical containers according to the present invention (with connection parts) in which port members are provided at both sides of side edges.

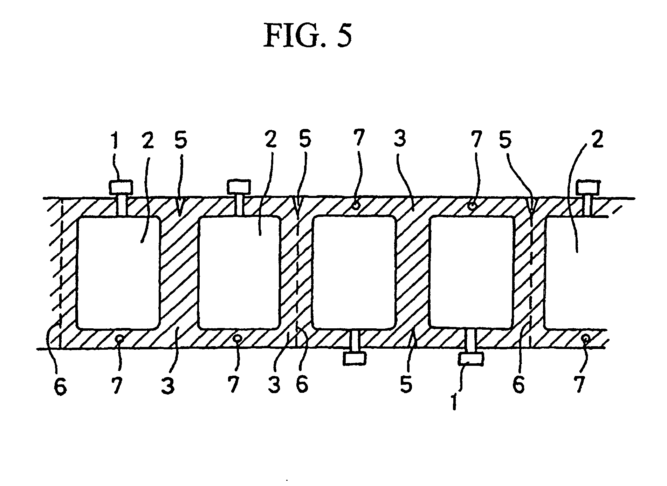

Fig. 5 shows multiple connected medical containers according to the present invention in which a port member is provided at one side of side edges

### BEST MODE FOR CARRYING OUT THE INVENTION

The present invention provides multiple connected medical containers in which two continuous films are sealed with each other by melting, bag type compartments are continuously formed, and a port member is provided at one side or both sides of side edges of each bag type compartment. Since the container is formed in a manner such that the entire bag-forming process, including sealing of the port members, is linked so that the continuous piece of film does not need to be cut, the structure of the bag-manufacturing equipment can be simplified, the ability to respond to changes in container embodiments is improved, adjustments are made easier, manufacturing costs are decreased as a result of a reduction in the area for facilities, introduction of foreign matter is prevented, packaging is easy, load shift during relay does not occur, and continuous and automated feeding of the containers to the filling equipment is facilitated.

The film employed in these multiple connected medical containers may be a film tube that is formed by the usual inflation molding method, or a film formed by a T-die molding method. Two pieces of the film may be overlapped or a wide continuous piece of film may be folded over into two layers. In particular, it is preferable to employ inflation film which is in the form of tubular, with the inside of the tube being maintained in a sterile state from the time of film molding, so that, when molding the connected containers, the side edges are cut off and the container can then be used without further modification.

The aforementioned film may be a single layer. However, a laminate film of two or more layers is more typically employed, with it being preferable to employ a resin such as an ethylene or propylene derived polymer which can be melt sealed using heat or high frequency sealing for the inner surfaces of the film. The thickness of the film is optimally selected according to the container volume and its desired objectives, however, for transfusion fluid bags, a thickness of 100∼500 µm, and particularly 200∼400 µm, is typically employed.

The bag type compartments are formed by melt sealing the perimeter of a compartment at set intervals along the two layer film that was stacked as described above (in the present invention, the area at which melt sealing occurs to form the bag type compartiments is referred to as the "jointing part of compartments"). In this case, the bags can be formed with a continuous piece of film by melting port members to one or both side edges of the film along its continuous direction, except making many sheets. In the contents filling process, as well as in packaging and taking out, the connected containers can be handled as a unit. Furthermore, if possible, the containers may also be designed to enable storage of a variety of different medical solutions. Namely, a partition can be provided inside the bag type compartments using a weak seal, to permit separate storage of different medicinal agents that are preferably not maintained in a mixed together state over a long period of time. Then, at the time of use, the weak seal can be broken by applying pressure, allowing the partitions to communicate, so that the medicinal solutions can mix together and then be used promptly.

The port members may be equivalent to that employed in transfusion fluid bags and the like. When attaching the port members, it is preferable that every set number of bag type compartments are folded without the port members stacking on each other when connected containers having the continuous bag type compartments as described above are folded after every set number of the port members to the number of folds.

It is preferable to minimize as much as possible the effect of the thickness of the port members by, for example, staggering where the folding portion is located so that stacking of the port members can be avoided; placing the port members so that they are staggered from the center of the bag type compartments so that they do not stack on top of one another when the connected containers are folded; or by providing the port member to only one side edge of the bag type compartment for every set number of bags to suit the folded length of the containers when the connected containers are folded.

The port member serves as a port for filling the container with the medicinal solution and for removing the medicinal solution. However, once the container has been filled with the medicinal solution, then in most cases it is sufficient if there is just a medicinal solution outlet port. For example, in the case of a container for medical use in which one port is acceptable, then a port may be provided to both sides of side edges of each bag. The center portion of the bag type compartment is cut (i.e., parallel to the longitudinal direction of the film), and this cut portion may be employed as the medicinal solution filling port.

This film stacked in two layers is sealed and bag type compartments are formed in continuous arrangement. An adhesive agent may be employed as the sealing method. However, rather than using an organic adhesive, a melt sealing method is preferable as it is not a problem with respect to the environment, there is no danger of content contamination, and it offers high productivity and is beneficial from the perspective of costs. For example, the bag type compartment can be formed by using a heating means such as high frequency or a hot die to seal 3 to 4 of the edges to the jointing parts, excluding the region at which the port member for each compartment is attached.

The continuous bag type compartments that are formed along the continuous direction of the film are connected by means of continuous jointing parts of compartments. These jointing parts of compartments may be parts extending from the sealed area along the perimeter of adjacent bag type compartments, or may be jointing parts of compartments at the center of which a non-sealed connection part is provided.

Note that it is preferable that the width of the jointing parts that will become the folding areas be larger than the maximum diameter of the port member (i.e., the length of the largest portion along the continuous direction of the connected containers), as this enables the folding area to be freely chosen so that the port members do not stack on top of one another.

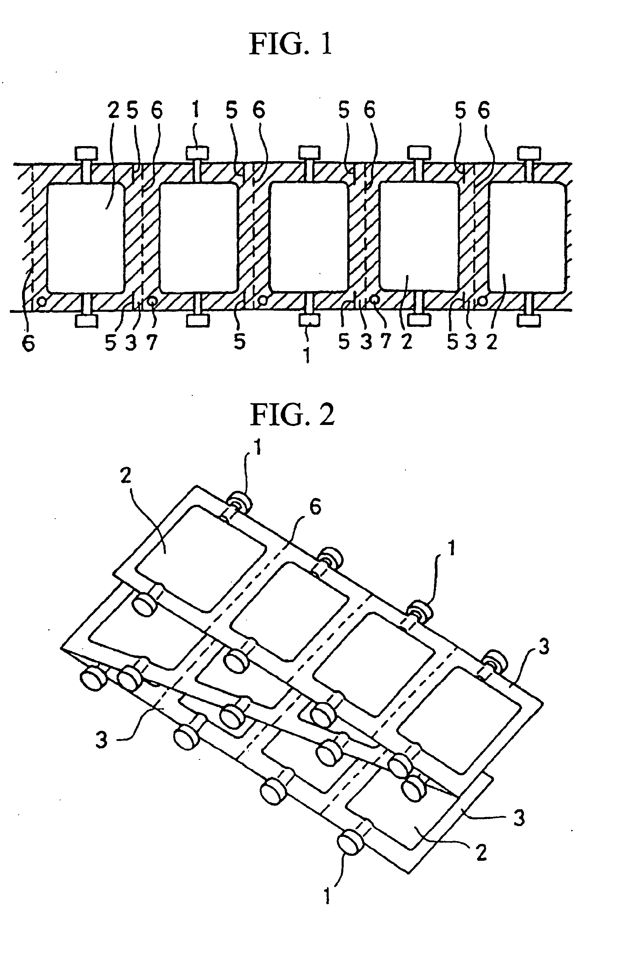

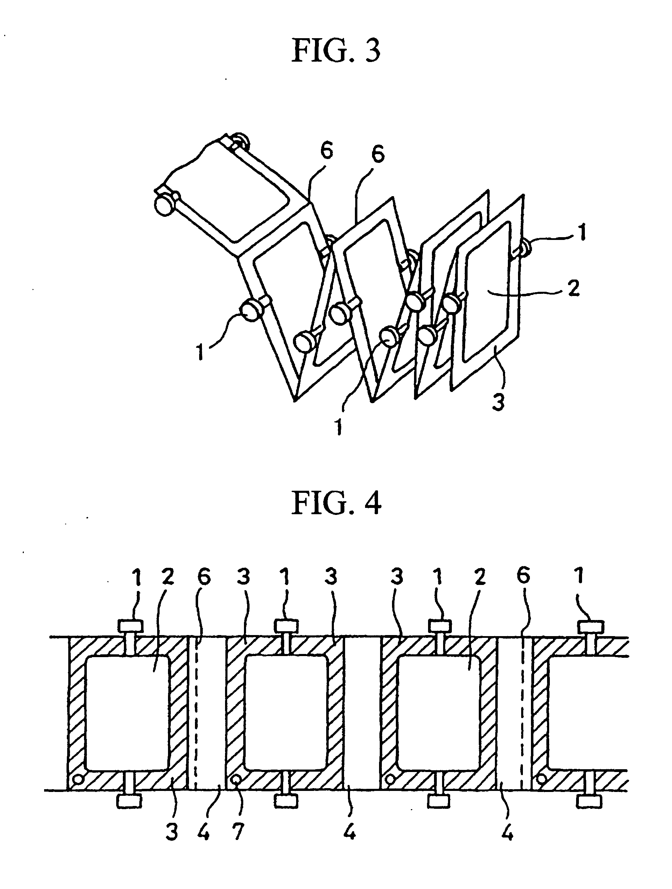

Since the length of the connected containers can be made endless, the connected containers need to be folded and handled in this manner during storage, packaging, and relay. In order to facilitate folding of the connected containers, a machine seam or interrupted slits may be provided to the jointing part of compartment or connection part at the outer periphery of the container, at the planned site for folding the containers. As a result, folding of the connected containers at the desired location is easily accomplished, improving workability. Methods such as shown in Figs. 2 and 3 are available as the folding method, which may be appropriately selected according to the circumstances.

In addition, the perforations or interrupted slits are useful for cutting free the compartments or for cutting free the each medical container. While it is not problematic if a portion of the aforementioned perforations or interrupted slits are common to the folding seam, it is also acceptable to provide separate folding seams to each compartment.

In these medical containers, when the port members, which have a self-supporting shape, are melted to the side edges of the continuous piece of film along the longitudinal direction of the film, the film in the melted area is not sufficiently long as compared to other areas due to the thickness of the port member. As a result, an excessive amount of tension is generated along the longitudinal direction of the two pieces of film, so that container deformation or positional deviation readily occurs, making it extremely difficult to maintain the connected containers in a flat state.

In order to handle connected medical containers in a flat state, it is necessary to carry out a means for releasing tension and strain at a suitable step either before or after attaching of the port member.

This means for releasing tension is not particularly restricted. For example, a notch may be introduced from the side edge of the connection part. Alternatively, stress or tension may be released by heating the jointing parts of compartments during or immediately after melt sealing, or by applying light tension after attaching of the port member so that the entire structure lies flat, and then heating near the side edges of the connected containers or heating the entire connected container. Further, another means that may be employed is one in which a connection part that is not melt sealed is provided between adjacent jointing parts of compartments when forming the bag type compartments. In addition, when releasing stress or stain by heating, it is acceptable to maintain a temperature distribution such that the relaxation portion does not have a narrow area.

The most preferable easing means is one in which a notch in the form of a slit, a V-shape or a U-shape is introduced to the jointing parts of compartments or the connection parts that are present on the sides where the port member is melted, this notch being introduced from the side edges running along the continuous direction of the bag type compartments, so that the side edges of the film can freely contract toward the port member.

In the case where a notch is introduced, it is beneficial to provide the notch before attaching the port member, as, in this case, unnecessary stress will not be applied on the film during melt sealing of the port member, so that an excellent seal can be achieved. The width and depth of the notch will vary depending on the size of the port member, the partitioning interval, film properties, port member melting conditions and the like. However, provided they are within limits that allow the connected containers to be handled in a flat state, then the width and depth dimensions may be freely selected. Stress is greatly released simply by providing a notch. It is preferable to provide a notch that has a depth equal to or slightly greater than the length of the port member (i.e., the length of the port member from the side edge entering into the bag), as this is effective in releasing stress on the side edges.

When employing the connected containers as transfusion fluid bags, it is useful to provide a hole for hanging the bag in the melt sealed portion opposite the outlet port.

The above-described multiple connected medical containers can be manufactured in the following way. Namely, while being intermittently or continuously fed, the film is melt sealed to form bag type compartments along its longitudinal direction, excluding the areas where the port members will be attached, the melt sealing occurring at each width of a bag type compartment, or at specific intervals which include a width dimension for the connection part. If necessary, the melted region can be rapidly cooled with a cooling die. In this case, it is acceptable to form adjacent bag type compartment so that they are in a state of contact, or a connection part which is not melt sealed may be provided in between adjacent bag type compartments.

Next, the area at which a port member will be attached is opened by pulling apart the film layers using a vacuum pad or the like. The port member is introduced through this opening, and a heated die is applied from outside the film to melt seal the port member in place.

In this case, because a relaxation means is performed before or after melting, it becomes possible to handle the containers in a flat state even though the containers are connected to one another.

In the case where equally sized port members are attached to both side edges, it is also very difficult to handle the connected containers in a flat state. Furthermore, if a large amount of stress is applied on the port just after it is sealed, the seal may tear away from the port. Accordingly, it is preferable to cool the area after melt sealing, however, the cooling can also cause greater stress to occur.

In particular, when only one port member is attached to a side edge, or when port members are provided to both side edges but are different in size (thickness), then it is nearly impossible to handle the connected containers in a flat state without further modification. From this perspective, if each bag is not cut away, then subsequent handling of the connected containers can often be impossible.

Stress at the side edges of the continuous piece of film is greatly released in connected containers in which a relaxation means has been executed. As a result, these connected containers can be handled in a flat manner (although the area at which the port members are located will significantly bulge) and peeling during sealing does not occur.

After attaching the port members, perforations or interrupted slits are provided at the predetermined folding site provided to the jointing parts of the bag type compartment or the connection parts.

Note that these molding sequences were provided as examples, and may be optionally varied to an extent that causes no problems in the processes performed.

Several of the connected containers manufactured in this way are organized into one row as shown in Figs. 2 and 3. Then, to fold the second row, a fold is made along the perforations or interrupted slits provided in the jointing part for the last container in row 1, so that the containers in row 2 lie on top of and are relatively displaced with respect to row 1, the amount of this displacement being equal to the width of the jointing part. As a result, the port members of the containers in the first and second rows can be aligned in a manner so as not to stack on top of one another. Next, by providing perforations or the like to the folding seam of the jointing part of the first container in the third row, and folding in a direction that is opposite row 2, row 3 can be folded in a manner similar to that described above, i.e., with a positional displacement so that the containers can be layered without any interference from the port members. The number of containers in each row is fixed. After a number of rows have been folded, the rows may be gradually layered inclined toward one direction, and, so that a port member does not become enclosed between adjacent containers in the same row, the number of containers in a row can be increased by one every set number of rows, so that the row is folded back again above the folding position for the first row. In the preceding method, a plurality of containers are continuously folded, so that stacking of the port members can be held to a minimum, making the packing volume compact.

In a container provided with a port member on just one side, stacking of the port members can be controlled by providing the port members to only the right side edge of the containers in the first row for example, and providing the port members to only the left side edge of the containers in the second row. Alternatively, if the position of the port members in the first and third rows (i.e., odd numbered rows) is staggered with respect to the position of the port members in the second and fourth rows (i.e., even numbered rows), stacking of the port members can be controlled so that the packing volume can be reduced.

As another method, the position of the folding seam can be alternated for each container to be between either the jointing parts of compartments or the connection parts. If the containers are then alternately folded along these folding seams, the port members will each be staggered by an amount equal to the width of the non-container portion. The second row is then layered in a similar manner without any interference from the port members. As a result, the containers can be layered with the port members tightly aligned.

However, in these layering methods, if the same surfaces are stacked opposing one another (i.e., front face to front face, back face to back face) and one side is the printed face, then it can be protected using a protective film that is applied either continuously over the surface, or only over the area of overlap.

The multiple connected medical containers according to the present invention can be packaged into a packing case while being folded with a plurality of containers in a continuous state. When the connected containers are continuously fed into the filling process and filled with the appropriate content, then, in the case of a roll-shaped package, as each roll unit ends, it is necessary to join the finishing end of that roll (which was at the center of the roll) to the starting end of the next roll. In the connected containers according to the present invention, however, the starting end of the packaging in one packaging unit is removed to the outside of the packing case to be accessible, and the remaining connected containers are packed into each packaging unit from the bottom of the packing case, so that the starting end and the finishing end of the connected containers can be taken out from the packing case at the same time.

In a folding package that is designed to enable the starting end at the time of packaging to be removed to the outside of the packing case (package unit), this starting end (i.e. the starting end at the time of packaging) is employed as the finishing end during feeding, and can be connected in advance to the starting end (i.e., the finishing end at the time of packaging) of the next packing unit to be employed. As a result, a plurality of packing units can be connected in advance, so that continuous filling of the containers can take place over a long period of time.

The connected containers according to the present invention can be employed in a role-shaped package. When wrapping into a role, however, the longer the wrapping and the longer the period of time that the containers are maintained in a tightly wrapped state, the greater the chance that a curling distortion will be introduced into the container. Moreover, care must be exercised since damage by load shift or positional displacements can more readily occur if the containers are wrapped into a loose roll.

In addition to the folding method described above, it is also possible to employ a cylindrical core around which the containers are continuously wrapped in a specific direction, as this method will also allow the connected containers to be handled in a flat state. In this case, it is necessary to set optimal conditions for the core diameter, the wrapping tension, and wrapping diameter after taking into consideration such factors as deformation of the container or its tendency to curl.

Unlike conventional medical use containers, the multiple connected medical containers according to the present invention are folded, and are packed in the connected state into a packing case. As a result, these containers can be supplied to the filling equipment in this connected state. For this reason, positioning of the container in the filling equipment is easily accomplished, making efficient processing possible.

It is also acceptable to cut free and pack each bag after the bag-forming process or the contents filling process. Alternatively, if the cutting free and packing is made by two or more bags, then the user can cut free each bag and freely select the product.

### Examples

Experiments employing the following methods were performed to test means for releasing the tension generated along the edge of the continuous film as a result of sealing a port member.

### Example 1

Straight-chain, low-density polyethylene monolayer inflation film having a width of 280 mm and a thickness of 300 µm was employed as the film starting roll.

First, the starting role, which is tightly wrapped into the shape of a roll, is drawn out to a specific interval intermittently, and a continuous cut is made with a knife-shaped cutter at a point approximately 10 mm from the side edge (ends) at which the port member is sealed. After making the film into two continuous stacked pieces and stopping, a notch is made with a scissor 40 mm from the end of the film at a position that is roughly in the center of the space interval between two port members (i.e., in a jointing part), and at a right angel with respect to the longitudinal direction of the continuous piece of film. This operation to introduce a notch is carried repeatedly each time a piece of film is relayed, either at the end of or at the same time as the next feed.

After introducing the notch, the two pieces of film that have been brought together are opened up using a vacuum pad, and a port member in which the seal portion has an outer diameter of 17 mm and a length of 15 mm (equal to the length of insertion of the port member into the film) is inserted. A sealing die which has been heated to a temperature of 150°C by a heater is then employed to hold the port from the outside of the film and heat it for approximately 3 seconds while applying pressure, thereby attaching the port member to the film. At the same time, jointing parts are simultaneously joined, to form the bag type compartments. The sealing die is opened, and the film is relayed again, this time to a water-cooled cooling die that is used to hold the film from either side and cool the sealed area. This operation is carried out repeatedly.

As a result, this film with the notches introduced in this manner did not experience deformations after sealing of the port member. Moreover, since it was cooled, peeling of the film away from the port member was not observed immediately after sealing of the port. Note that the notch had a V-shaped form under tension.

### Comparative Example 1

With the exception of providing a notch, a process equivalent to that of Example 1 was performed. In this case, the film would not stay flat after sealing of the port member, presenting a hindrance in the subsequent transport and packaging processes. In addition, the film pealed away from the port member due to tension generated after sealing of the port member. As a result, the containers were not air tight, making it impossible to obtain containers of the necessary quality for use as medical containers.

### Example 2

A piece of film equivalent to that in Example 1 was drawn out in the same manner as in that example, and port members were introduced and sealed. In this case, however, a metal die heated to 140°C was brought into contact for 2∼3 second with the ends of the film at adjacent jointing parts of compartments on either side over an area that was 20 mm in the longitudinal direction and 40 mm in the width direction of the film. As a result, the heated area was drawn out in the longitudinal direction of the film due to the stress generated by sealing of the port members. The same results were obtained as in Example 1.

### INDUSTRIAL APPLICABILITY

The present invention relates to bag type multiple connected medical containers in which a port member can be melted and attached continuous from the film, the container manufacturing process for these multiple connected medical containers being highly economical because of the simple machine equipment and small amount of facility space required. Even with the containers in the connected state, tension arising from melting of the port member is released in these containers, so that they can be handled as a flat connected unit, and folded packaging can be automated. Furthermore, packaged containers can be continuously fed to the contents filling equipment, and it is possible to greatly reduce the occurrence of such problems as positional displacements, falling, scratching, and the like. that can happen when the containers are individually clamped or delivered after having been sheets. Furthermore, these multiple connected medical containers are superior with respect to the release and surety with which the continuously supplied container can be packed, stored, taken out and separated into individual bags after filling. Of course, it is also acceptable to render individual bags by cutting free either before or after filling, and then filling, packaging, and storing the containers may be carried out.

## Claims

1. Multiple connected medical containers comprising: a continuous film having a melted sealed part; bag type compartments which are continuously provided by the melted sealed part; a port member which is provided at one side or both sides of side edges of each bag type compartment; and a relaxation means for releasing stress or tension at side edges of the continuous film.

2. Multiple connected medical containers according to claim 1, wherein the relaxation means is a jointing part of compartments or a connection part, each having a notch.

3. Multiple connected medical containers according to claim 2, wherein the notch is a slit, a V-shaped notch, or a U-shaped notch.

4. Multiple connected medical containers according to claim 1, wherein the relaxation means releases stress or tension by heating the side edges before or after providing the port member.

5. Multiple connected medical containers according to claim 1, wherein the relaxation means is a connection part which is provided between adjacent jointing parts of compartments without melting and sealing.

6. Multiple connected medical containers according to claim 1, further comprising a folding seam on the jointing part of compartments or the connection part so that every set number of bag type compartments is folded without the port members stacking on each other.

7. Multiple connected medical containers according to claim 6, wherein the jointing part of compartments has a width greater than a maximum diameter of the port member.

8. Multiple connected medical containers according to claim 1, wherein the port members are disposed toward the side edge of the bag type compartment so that every set number of bag type compartments is folded without the port members stacking on each other.

9. Multiple connected medical containers according to claim 1, wherein the port members are provided on alternating sides after every set number of the port members to the number of folds so that every set number of bag type compartments are folded without the port members stacking on each other.

10. Multiple connected medical containers according to claim 1, wherein the folding seam has perforations, notches, or discontinuous slits provided on the jointing part of compartments or the connection part.

11. Multiple connected medical containers according to claim 1, wherein the continuous film is in a form of tubular, a laminate of two films, or a two stacked layers film provided by folding a film having a wide width.

12. Multiple connected medical containers according to claim 1, wherein the continuous film is a plastic film being a single layer or a laminate with a thickness of 100 ∼ 500 µm.

13. Multiple connected medical containers according to claim 1, wherein the continuous film contains joining faces made by a melt sealable resin.

14. Multiple connected medical containers according to claim 1, used for continuous transfusion fluid bags or CAPD bags.

15. Multiple connected medical containers according to claim 1, comprising a hole for suspension provided at the melted sealed part of either port member.

16. A packing method of multiple connected medical containers, comprising a step of: folding the continuous multiple connected medical containers according to any one of claims 1 to 15 by a set number of two or more bag type compartments without the port members stacking on each other.

17. A packaging method of multiple connected medical containers, comprising a step of: packing the multiple connected medical containers so that compartments at a starting end and a finishing end in the multiple connected medical containers are taken out of the packaging unit, when the multiple connected medical containers according to claim 1 are packed into a packaging case (packaging unit) while being folded from a bottom of the case in order.

18. A manufacturing method of multiple connected medical containers, comprising steps of:
supplying two stacked pieces of a film to a melt sealer;
sealing by melting a jointing part of a compartment except for an attachment part of a port member;
pulling apart one or both side edges of the film where the attachment part of a port member is provided;
sealing by melting the port member; and
providing a notch at the side edges of the jointing part of compartments either before or after sealing by melting the port member.

19. A manufacturing method of multiple connected medical containers, comprising steps of:
supplying two stacked pieces of a film to a melt sealer;
sealing by melting a jointing part of a compartment except for an attachment part of a port member;
pulling apart one or both side edges of the film where the attachment part of a port member is provided;
forming multiple connected containers by melting and sealing the port member; and
heating the side edges or the entirety of the containers to relieve tension.

20. A contents filling method of multiple connected medical containers, comprising steps of:
supplying the multiple connected medical containers according to any one of claims 1 to 15 to a contents filling device continuously; and
filling contents into the multiple connected medical containers.
